# EUROPEAN PATENT SPECIFICATION

(11) **EP 4 312 739 B1**
(45) Date of publication and mention of the grant of the patent: **09.12.2026**
(21) Application number: 22773833.3
(22) Date of filing: 22.03.2022
(51) Int. Cl.: A61B 5/02, A61B 5/0205, A61B 5/318, A61B 5/0537

(54) **ASSESSMENT OF CARDIOVASCULAR FUNCTION THROUGH CONCOMITANT ACQUISITION OF ECG AND BIA**
BEURTEILUNG DER KARDIOVASKULÄREN FUNKTION DURCH GLEICHZEITIGE ERFASSUNG VON EKG UND BIA
ÉVALUATION DE LA FONCTION CARDIOVASCULAIRE PAR L'INTERMÉDIAIRE DE L'ACQUISITION CONCOMITANTE D'ECG ET DE BIA

(30) Priority: 22.03.2021 US 202163164199 P
(43) Date of publication of application: 07.02.2024
(73) Proprietor: The Royal Institution for the Advancement of Learning / McGill University, Montreal, Quebec H3A 0G4 (CA)
(72) Inventor: AFILALO, Jonathan, Québec H3X 3B4 (CA)
(74) Representative: Fiorucci, Hélène
(86) International application number: PCT/CA2022/050424
(87) International publication number: WO 2022/198312

(56) References cited:
- WO-A1-2022/256492
- JP-A- S 555 649
- JP-B2- 4 985 445
- US-A1- 2005 255 491
- US-A1- 2018 177 412
- US-B1- 10 998 101
- US-B2- 10 532 211
- SHIN SEUNG-CHUL ET AL: "Two electrode based healthcare device for continuously monitoring ECG and BIA signals", 2018 IEEE EMBS INTERNATIONAL CONFERENCE ON BIOMEDICAL & HEALTH INFORMATICS (BHI), IEEE, 4 March 2018 (2018-03-04), pages 141 - 144, XP033345099, DOI: 10.1109/BHI.2018.8333389

## Description

### CROSS-REFERENCE TO RELATED APPLICATIONS

The present application claims priority of U.S. Provisional Patent Application No. 63/164,199, filed on March 22, 2021, q.v..

### TECHNICAL FIELD

The disclosure generally relates to the field of cardiovascular assessment through ECG and BIA.

### BACKGROUND OF THE ART

An electrocardiogram (ECG) records the electrical activity of the heart and provides information about heart rate, rhythm, and disease. Bioelectrical impedance analysis (BIA) records the electrical impedance of the body and provides information about body composition, in particular body water. There exist many different types of systems for performing ECG and other systems for performing BIA. While these systems are suitable for their purposes, improvements are desired. US 2018/177412 A1 discloses a medical measuring device.

### SUMMARY

In accordance with one aspect, there is provided a system for assessing cardiovascular functions of a patient. The system comprises a processor and a non-transitory computer-readable medium having stored thereon program instructions. The program instructions are executable by the processor for acquiring ECG data and BIA data concomitantly through a plurality of electrodes positioned in an ECG configuration, predicting parameters of cardiovascular function from the ECG data and the BIA data using deep learning algorithms, and outputting surrogates of parameters of cardiovascular function in a clinical format.

In some embodiments, predicting the parameters of cardiovascular function comprises generating impedance plethysmography (IPG) data representative of cardiovascular function over time from the ECG data and BIA data.

In some embodiments, predicting the parameters of cardiovascular function comprises generating impedance tomography (ITG) data, comprising static or dynamic images of a heart and a lung of the patient, representative of cardiovascular and pulmonary function over time from the ECG data and BIA data.

In some embodiments, predicting the parameters of cardiovascular function comprises predicting a level or proxy of brain natiuretic peptide (BNP) or N-terminal brain natiuretic peptide (NT-proBNP) to represent a likelihood and associated severity of a diagnosis of heart failure, whereby severity informs a prognosis and treatment of the patient.

In some embodiments, predicting the parameters of cardiovascular function comprises predicting a level or proxy of central venous pressure (CVP) similar to right atrial pressure (RAP) to represent a likelihood and associated severity of a diagnosis of heart failure, whereby severity informs a prognosis and treatment of the patient.

In some embodiments, predicting the parameters of cardiovascular function comprises predicting a level or proxy of pulmonary capillary wedge pressure (PCWP) similar to left atrial pressure (LAP) to represent a likelihood and associated severity of a diagnosis of heart failure, whereby severity informs a prognosis and treatment of the patient.

In some embodiments, predicting the parameters of cardiovascular function comprises predicting a level or proxy of ventricular ejection fraction (VEF) similar to systolic function to represent a likelihood and associated severity of a diagnosis of heart failure, whereby severity informs a prognosis and treatment of the patient.

In some embodiments, predicting the parameters of cardiovascular function comprises predicting a level or proxy of late gadolinium enhancement (LGE) similar to myocardial viability to represent a likelihood and associated severity of a diagnosis of heart failure, whereby severity informs a prognosis and treatment of the patient.

In some embodiments, predicting the parameters of cardiovascular function comprises predicting a level or proxy of pulmonary venous congestion to represent a likelihood and associated severity of a diagnosis of pulmonary edema, whereby severity informs a prognosis and treatment of the patient.

In some embodiments, predicting the parameters of cardiovascular function comprises predicting a level or proxy of segmental venous congestion to represent a likelihood and associated severity of a diagnosis of deep venous occlusion or peripheral edema, whereby severity informs a prognosis and treatment of the patient.

In some embodiments, predicting the parameters of cardiovascular function comprises predicting a level or proxy of ankle brachial index (ABI) to represent a likelihood and associated severity of a diagnosis of peripheral arterial occlusion, whereby severity informs a prognosis and treatment of the patient.

In some embodiments, the program instructions are further executable by the processor for applying the deep learning algorithms for modeling changes in the parameters of cardiovascular function to support pharmacologic treatment decisions and monitor treatment effects.

In some embodiments, the program instructions are further executable by the processor for presenting one or more suggestions for administration and tailored dosage of intravenous fluids in accordance with an anticipated hemodynamics responsiveness based on the modeling of the changes in the parameters of cardiovascular function.

In some embodiments, the program instructions are further executable by the processor for presenting one or more suggestions for administration and tailored dosage of diuretic drugs based on the modeling of the changes in the parameters of cardiovascular function.

In some embodiments, the program instructions are further executable by the processor for applying the deep learning algorithms for modeling changes in parameters of body composition and pharmacokinetics to support pharmacologic treatment decisions and monitor treatment effects.

In some embodiments, the program instructions are further executable for predicting a level or proxy of hydrophilic drug concentrations in a body of the patient and suggestions for tailored dosage of hydrophilic drugs based on the modeling of the changes in the parameters of body composition and pharmacokinetics.

In some embodiments, the hydrophilic drugs comprise anticoagulant drugs and/or chemotherapy drugs.

In some embodiments, the program instructions are further executable by the processor for applying the deep learning algorithms for detecting a likelihood of heart failure or cardiotoxicity and an associated severity.

In some embodiments, the program instructions are further executable by the processor for applying the deep learning algorithms for detecting a likelihood of frailty and an associated severity.

In some embodiments, the program instructions are further executable by the processor for predicting a likelihood of ancillary comorbid diagnoses having a correlation with the likelihood of frailty.

In some embodiments, the program instructions are further executable by the processor for predicting a likelihood of future adverse health events using at least one of the likelihood of heart failure and the likelihood of frailty.

In some embodiments, the program instructions are further executable by the processor for predicting a probability of death, heart failure related decompensation, readmission, or other adverse health events.

In some embodiments, the program instructions are further executable by the processor for outputting a measure of cardio-geriatric risk that reflects cumulative cardiac and geriatric impairments.

In some embodiments, the program instructions are further executable by the processor for presenting one or more suggestions for optimization of care to therapeutically target future adverse health events identified.

In some embodiments, the program instructions are further executable by the processor for predicting a readiness for hospital discharge or a need for hospital admission.

In accordance with another aspect, there is provided a method for assessing cardiovascular functions of a patient. The method comprises acquiring ECG data and BIA data concomitantly through a plurality of electrodes positioned in an ECG configuration, predicting parameters of cardiovascular function from the ECG data and the BIA data using deep learning algorithms, and outputting surrogates of parameters of cardiovascular function in a clinical format.

In some embodiments, predicting the parameters of cardiovascular function comprises generating impedance plethysmography (IPG) data representative of cardiovascular function over time from the ECG data and BIA data.

In some embodiments, predicting the parameters of cardiovascular function comprises generating impedance tomography (ITG) data, including static or dynamic images of a heart and a lung of the patient, representative of cardiovascular and pulmonary function over time from the ECG data and BIA data.

In some embodiments, predicting the parameters of cardiovascular function comprises predicting a level or proxy of brain natiuretic peptide (BNP) or N-terminal brain natiuretic peptide (NT-proBNP) to represent a likelihood and associated severity of a diagnosis of heart failure, whereby severity informs a prognosis and treatment of the patient.

In some embodiments, predicting the parameters of cardiovascular function comprises predicting a level or proxy of central venous pressure (CVP) similar to right atrial pressure (RAP) to represent a likelihood and associated severity of a diagnosis of heart failure, whereby severity informs a prognosis and treatment of the patient.

In some embodiments, predicting the parameters of cardiovascular function comprises predicting a level or proxy of pulmonary capillary wedge pressure (PCWP) similar to left atrial pressure (LAP) to represent a likelihood and associated severity of a diagnosis of heart failure, whereby severity informs a prognosis and treatment of the patient.

In some embodiments, predicting the parameters of cardiovascular function comprises predicting a level or proxy of ventricular ejection fraction (VEF) similar to systolic function to represent a likelihood and associated severity of a diagnosis of heart failure, whereby severity informs a prognosis and treatment of the patient.

In some embodiments, predicting the parameters of cardiovascular function comprises predicting a level or proxy of late gadolinium enhancement (LGE) similar to myocardial viability to represent a likelihood and associated severity of a diagnosis of heart failure, whereby severity informs a prognosis and treatment of the patient.

In some embodiments, predicting the parameters of cardiovascular function comprises predicting a level or proxy of pulmonary venous congestion to represent a likelihood and associated severity of a diagnosis of pulmonary edema, whereby severity informs a prognosis and treatment of the patient.

In some embodiments, predicting the parameters of cardiovascular function comprises predicting a level or proxy of segmental venous congestion to represent a likelihood and associated severity of a diagnosis of deep venous occlusion or peripheral edema, whereby severity informs a prognosis and treatment of the patient.

In some embodiments, predicting the parameters of cardiovascular function comprises predicting a level or proxy of ankle brachial index (ABI) to represent a likelihood and associated severity of a diagnosis of peripheral arterial occlusion, whereby severity informs a prognosis and treatment of the patient.

In some embodiments, the method further comprises applying the deep learning algorithms for modeling changes in the parameters of cardiovascular function to support pharmacologic treatment decisions and monitor treatment effects.

In some embodiments, the method further comprises presenting one or more suggestions for administration and tailored dosage of intravenous fluids in accordance with an anticipated hemodynamics responsiveness based on the modeling of the changes in the parameters of cardiovascular function.

In some embodiments, the method further comprises presenting one or more suggestions for administration and tailored dosage of diuretic drugs based on the modeling of the changes in the parameters of cardiovascular function.

In some embodiments, the method further comprises applying the deep learning algorithms for modeling changes in parameters of body composition and pharmacokinetics to support pharmacologic treatment decisions and monitor treatment effects.

In some embodiments, the method further comprises predicting a level or proxy of hydrophilic drug concentrations in a body of the patient and suggestions for tailored dosage of hydrophilic drugs based on the modeling of the changes in the parameters of body composition and pharmacokinetics.

In some embodiments, the hydrophilic drugs comprise anticoagulant drugs and/or chemotherapy drugs.

In some embodiments, the method further comprises applying the deep learning algorithms for detecting a likelihood of heart failure or cardiotoxicity and an associated severity.

In some embodiments, the method further comprises applying the deep learning algorithms for detecting a likelihood of frailty and an associated severity.

In some embodiments, the method further comprises predicting a likelihood of ancillary comorbid diagnoses having a correlation with the likelihood of frailty.

In some embodiments, the method further comprises predicting a likelihood of future adverse health events using the likelihood of heart failure and the likelihood of frailty.

In some embodiments, the method further comprises predicting a probability of death, heart failure related decompensation, readmission, or other adverse health events.

In some embodiments, the method further comprises outputting a measure of cardio-geriatric risk that reflects cumulative cardiac and geriatric impairments.

In some embodiments, the method further comprises presenting one or more suggestions for optimization of care to therapeutically target future adverse health events identified.

In some embodiments, the method further comprises predicting a readiness for hospital discharge or a need for hospital admission.

Many further features and combinations thereof concerning embodiments described herein will appear to those skilled in the art following a reading of the instant disclosure.

### DESCRIPTION OF THE FIGURES

In the figures,
Figs. 1A-1D are schematic examples of medical systems for monitoring cardiovascular function;
Figs. 2A-2E illustrate various examples of ECG configurations;
Figs. 3A-3C are example outputs of the system of Figs. 1A-1D;
Fig. 4 is a flowchart of an example method for assessing cardiovascular function;
Fig. 5 is a schematic of an example analytical pipeline for assessing cardiovascular function; and
Fig. 6 is a block diagram of an example computing device.

### DETAILED DESCRIPTION

The present disclosure is directed to methods and systems for assessing cardiovascular functions of a patient by processing electrocardiogram (ECG) and bioelectric impedance analysis (BIA) data acquired concomitantly using an ECG electrode configuration. ECG is one of the most commonly performed diagnostic tests in medicine; used to describe the heart's intrinsic electrical activity in order to help diagnose a wide variety of cardiac conditions. BIA is used to describe the body's composition, and more specifically, the distribution of water and estimated lean mass and fat mass of the body. Together, ECG and BIA are used for enhanced heart failure diagnosis that provides support for individualized treatment and prognostication.

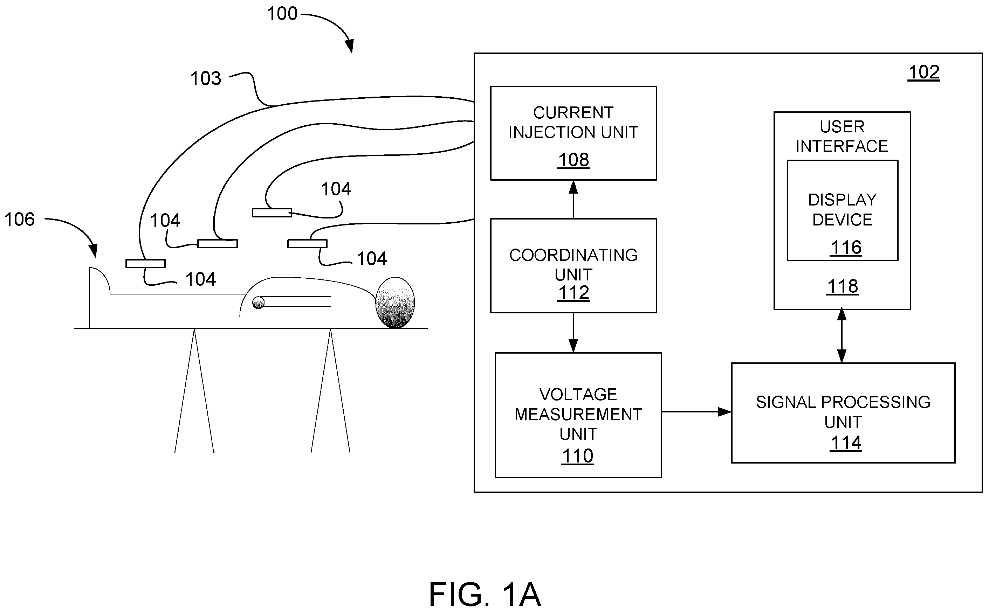

With reference to Fig. 1A, there is illustrated a first example of a medical system 100 for patient monitoring. A monitoring device 102 is coupled to a plurality of electrodes 104 connectable to the body of a patient 106 in an ECG configuration. Only four electrodes 104 are illustrated but more or less may be provided. The patient 106 is shown to be in the supine position but may alternatively be positioned differently. The electrodes 104 act as signal-measuring electrodes, signal-injecting electrodes, or both, as will be explained in more detail below. In the embodiment of Fig. 1A, the electrodes 104 are coupled to the monitoring device 102 via a plurality of cables 103 but could alternatively be coupled using various wireless means, such as but not limited to Bluetooth, Zigbee, Radio Frequency Identification (RFID), and the like. The electrodes 104 may be wet electrodes or dry electrodes, and the dry electrodes may be contact or noncontact electrodes.

The monitoring device 102 is configured for coordinating the concomitant acquisition of ECG measurements and BIA measurements through the same ECG configuration. In order to obtain the ECG measurements, a voltage measurement unit 110 performs passive measurements between pairs of electrodes 104 to capture the heart's electrical signals, by measuring the difference in electric potential of a given pair of electrodes 104 (which may include one or more virtual electrodes). In some embodiments, a third one of the electrodes 104 is used to cancel out a common mode noise when performing the voltage measurement across two other ones of the electrodes 104. In order to obtain BIA measurements, a current injection unit 108 applies current to pairs of electrodes 104 to create one or more conduction path in the body. The BIA measurements are then obtained by performing active measurements via the voltage measurement unit 110 across pairs of electrodes 104 that lie within a conductive path. For the purposes of the present disclosure, passive measurements are for ECG and active measurements are for BIA.

A coordinating unit 112 controls current injection and voltage measurement for the monitoring device 102 to acquire both BIA and ECG data using the same ECG configuration. This information about the patient 106 can be obtained within one sitting (i.e. during a single test) and also facilitates the use of the monitoring device 102 for technicians and operators who are already familiar with the traditional ECG test. The acquisition of the BIA measurements concomitantly with the ECG measurements is thus performed transparently to the operator of the monitoring device 102.

In some embodiments, the coordinating unit 112 is configured to obtain the ECG data and the BIA data sequentially. That is to say, all passive measurements are performed by the voltage measurement unit 110 and once the passive measurements are completed, the current injection unit 108 creates the conduction paths and the voltage measurement unit 110 performs active measurements. The reverse order may also be used.

In some embodiments, the coordinating unit 112 is configured to obtain the ECG measurements and the BIA measurements in a series of alternating sequences. For example, a first sequence of passive measurements may be followed by a first sequence of active measurements which may be followed by a second sequence of passive measurements, and so on. One or more measurement may be performed during each sequence.

In some embodiments, the coordinating unit 112 is configured to obtain the ECG measurements and the BIA measurements concurrently, using one or more pre-determined measurement patterns that may be stored in the monitoring device 102 or remotely therefrom. For example, depending on the ECG configuration used, it may be possible to apply current across a first pair of electrodes 104 and measure voltage across a second pair of electrodes 104 that lies within the conductive path between the first pair of electrodes 104 while also measuring voltage across a third pair of electrodes 104. In some embodiments, the measurement pattern may depend on the ECG electrode configuration, the test performed, the test time, the desired output, and other parameters affecting the ability to perform ECG and BIA measurements concurrently. Coordination is managed by the coordinating unit 112, in accordance with a given measurement pattern that dictates where to inject current, where to measure voltage, and using what sequence.

Captured voltage measurements are provided to a signal processing unit 114 and an output is displayed on a display device 116, which may form part of a user interface 118. Although illustrated as part of the monitoring device 102, the display device 116 and/or user interface 118 may also be provided separately therefrom. In some embodiments, an operator may enter information on the patient 106 via the user interface 118 and this information may be used in conjunction with the measured data to produce an output. In some embodiments, an operator may be asked to make one or more selections regarding the test to be performed, the ECG configuration, and the desired output via the user interface 118 and this information may be used in conjunction with the measured data to produce an output.

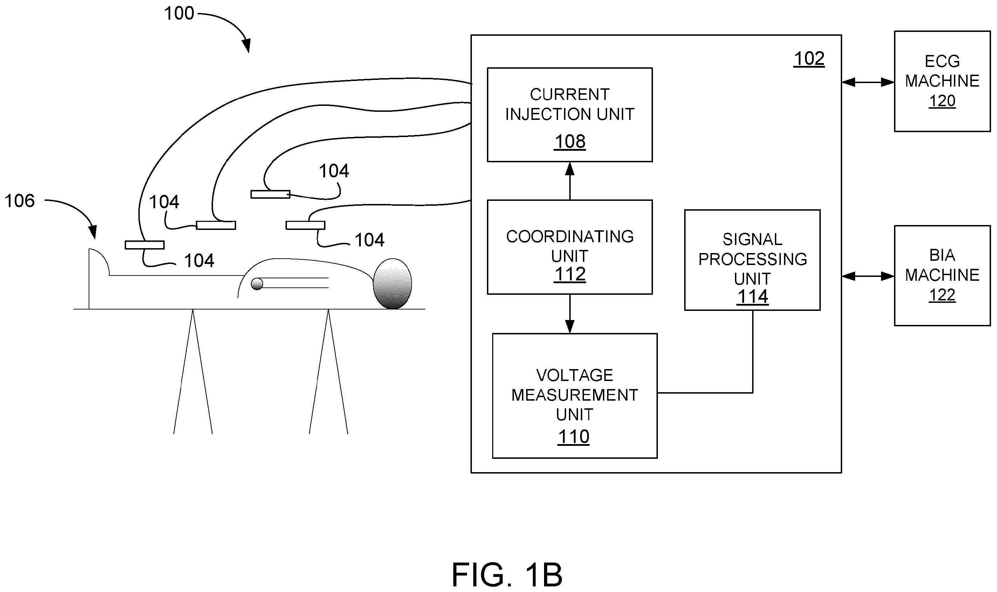

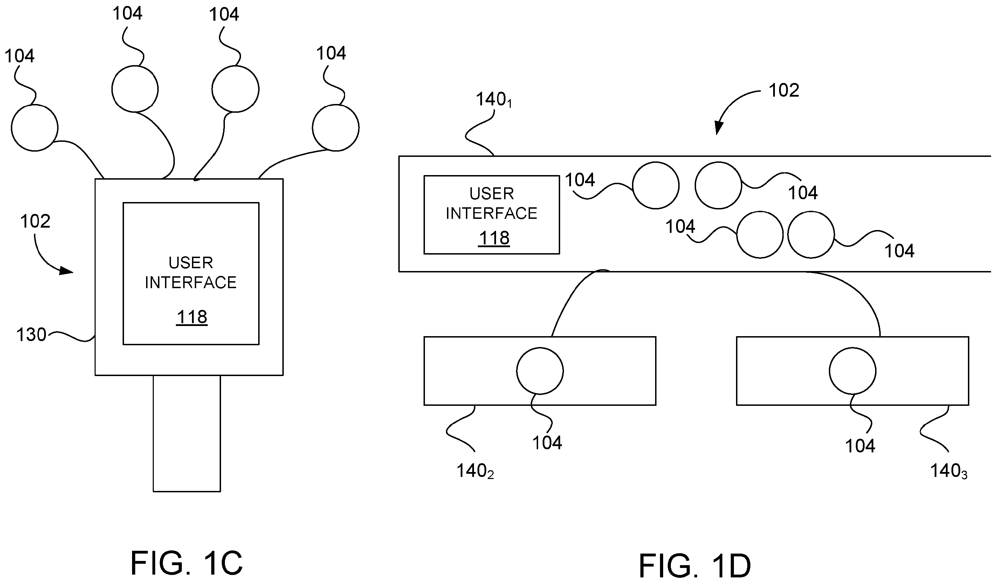

As shown in Fig. 1A, the monitoring device 102 may be a standalone machine with built-in ECG and BIA signal acquisition capabilities that connects to electrodes 104. For example, the monitoring device 102 may be implemented on an electronic circuit board supporting various electronic components including, but not limited to, a first chip that receives input from one or more of the electrodes 104 and implements the ECG signal acquisition capabilities to generate ECG data, a second chip that receives input from one or more of the electrodes 104 and implements the BIA signal acquisition capabilities to generate BIA data, and a microprocessor that processes the ECG data and the BIA data and generates at least one output based thereon. One or more relays may be used (e.g., by redirecting one or more of the electrodes 104) to connect the ECG circuitry provided in the first chip to the BIA circuitry provided in the second chip. In another embodiment, shown in Fig. 1B, the monitoring device 102 is a companion unit insertable between the electrodes 104 and an ECG machine 120 and/or a BIA machine 122. The user interface 118 and/or display 116 may be part of the monitoring device 102 or an existing user interface/display device from the ECG machine 120 and/or BIA machine 122 may instead be used. In some embodiments, as shown in Fig. 1C, the monitoring device 102 is a handheld unit 130 having electrodes 104 integrated therein or attached thereto. In yet another embodiment, as shown in Fig. 1D, the monitoring device 102 is a portable or wearable unit, formed of one or more components 140₁, 140₂, 140₃ attachable directly to the body, such as the chest (e.g. 140₁) and the limbs (e.g. 140₂, 140₃). Other embodiments are also contemplated, for example as a scale (i.e. weighing device) with electrodes embedded in the placeholders for the feet and hand supports, or as an elongated member (e.g., a bar, tube or the like) having two opposite ends and one or more electrodes provided at each end .

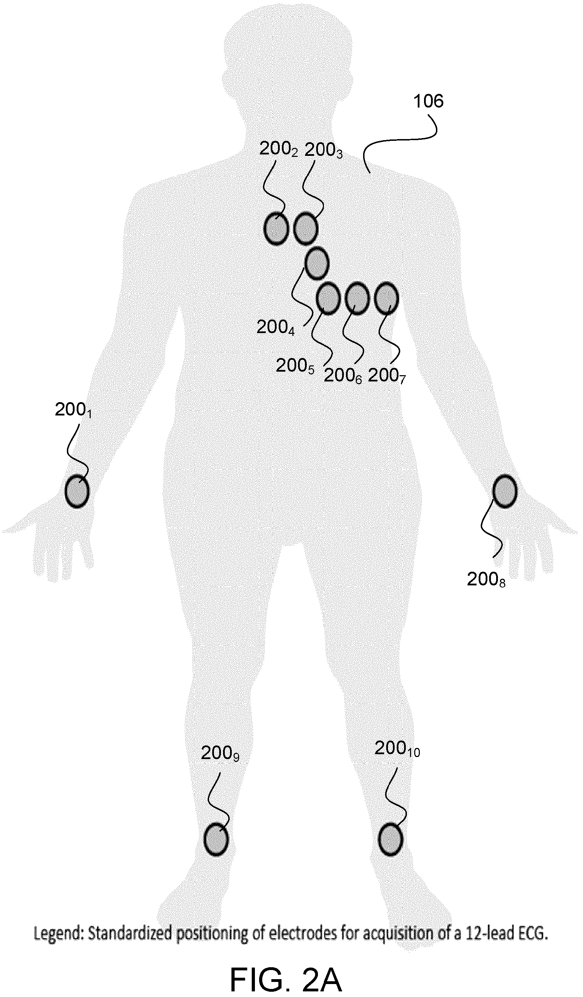

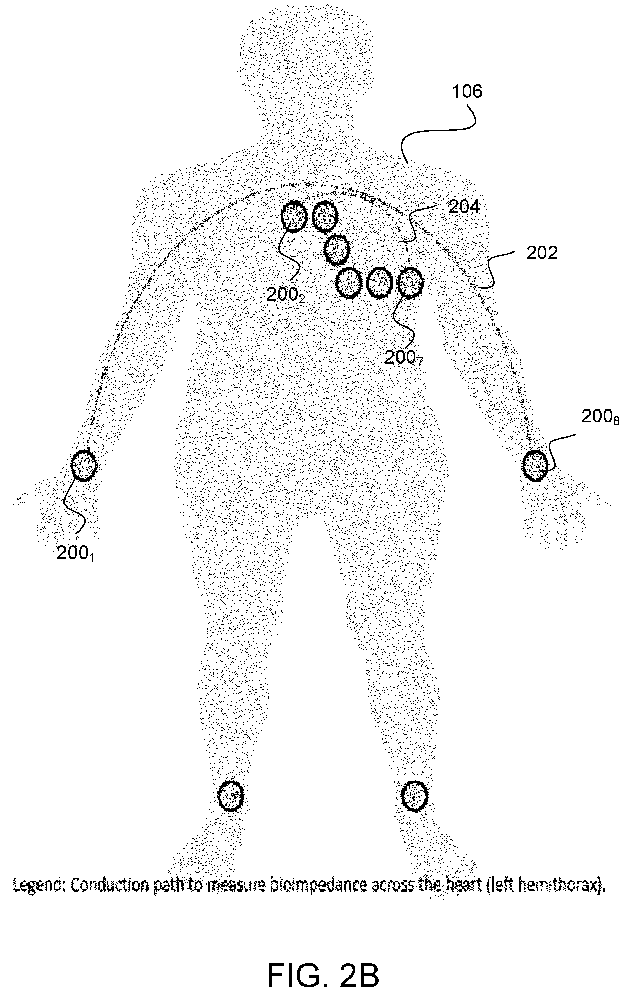

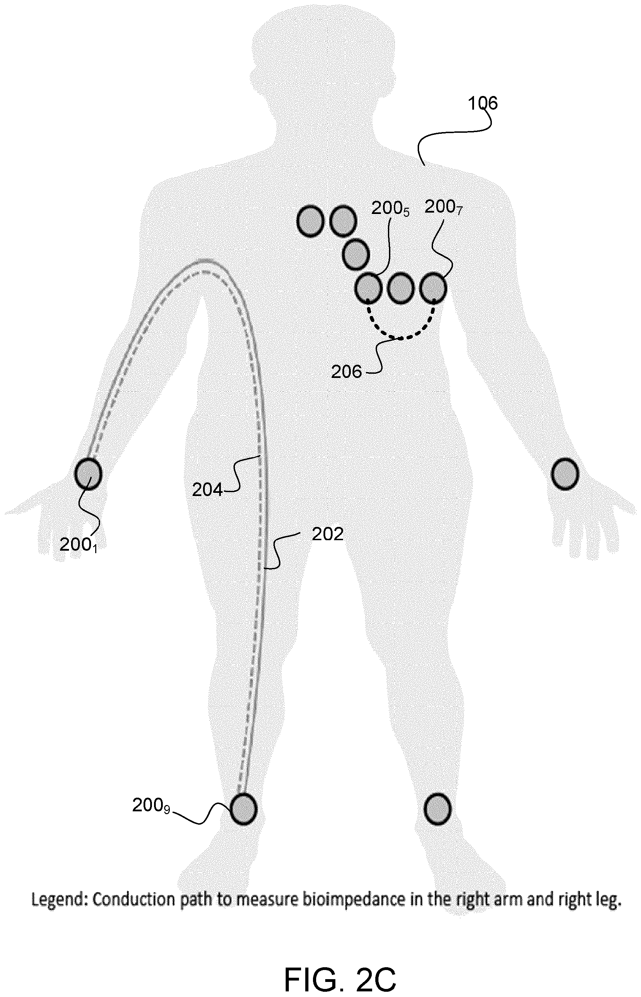

In some embodiments, the ECG configuration from which the ECG and BIA data is captured is a standard 12-lead configuration. With reference to Fig. 2A, an example is illustrated for the electrode positioning in the standard 12-lead configuration, whereby ten electrode positions 200₁ - 200₁₀ are located on the body of the patient 106 such that there are electrodes on each limb at electrode positions 200₁, 200₈, 200₉, 200₁₀ respectively, and on the chest at six precordial electrode positions 200₂ - 200₇. Fig. 2B illustrates an example for acquiring BIA data using the standard 12-lead ECG configuration. Current is injected across a pair of electrodes at positions 200₁ and 200₈ to create conduction path 202. Active voltage measurements are taken across electrodes located at electrode positions 200₂ and 200₇ along voltage measurement path 204 that lies within the conduction path 202. Fig. 2C illustrates an example for acquiring BIA measurements and ECG measurements concurrently, using the standard 12-lead ECG configuration. Current is injected across a pair of electrodes at positions 200₁ and 200₉ to create conduction path 202. Active voltage measurements are taken across electrodes located at electrode positions 200₁ and 200₉ along voltage measurement path 204 that lies within the conduction path 202. Passive voltage measurements are taken across electrodes located at electrode positions 200₅ and 200₇ along voltage measurement path 206. In this example, the active measurements (for BIA) and passive measurements (for ECG) may be taken concurrently. Passive voltage measurements may also be taken concurrently with active voltage measurements across electrodes that lie within the conduction path 202 using various filtering techniques that can isolate the ECG voltage measurements from the BIA voltage measurements.

Various electrode configurations are contemplated. For example, two separate electrodes may be positioned side by side at electrode position 200₁ such that a first of the two electrodes is a signal-injecting electrode and a second of the two electrodes is a signal-measuring electrode. In another example, a same electrode may be used as both a signal-injecting electrode and a signal-measuring electrode. In yet another example, a same electrode may be subdivided such that the one part is signal-injecting and another part is signal-measuring. Therefore, in some embodiments of the 12-lead ECG electrode configuration, all electrodes can be signal-injecting electrodes and signal-measuring electrodes and there are 10 electrodes. In some embodiments of the 12-lead ECG electrode configuration, 10 electrodes are signal-measuring electrodes, 2 electrodes are signal-injecting electrodes, and there are 12 electrodes. In some embodiments of the 12-lead ECG electrode configuration, 10 electrodes are signal-measuring electrodes, 4 electrodes are signal-injecting electrodes, and there are 14 electrodes. Other embodiments may also be used.

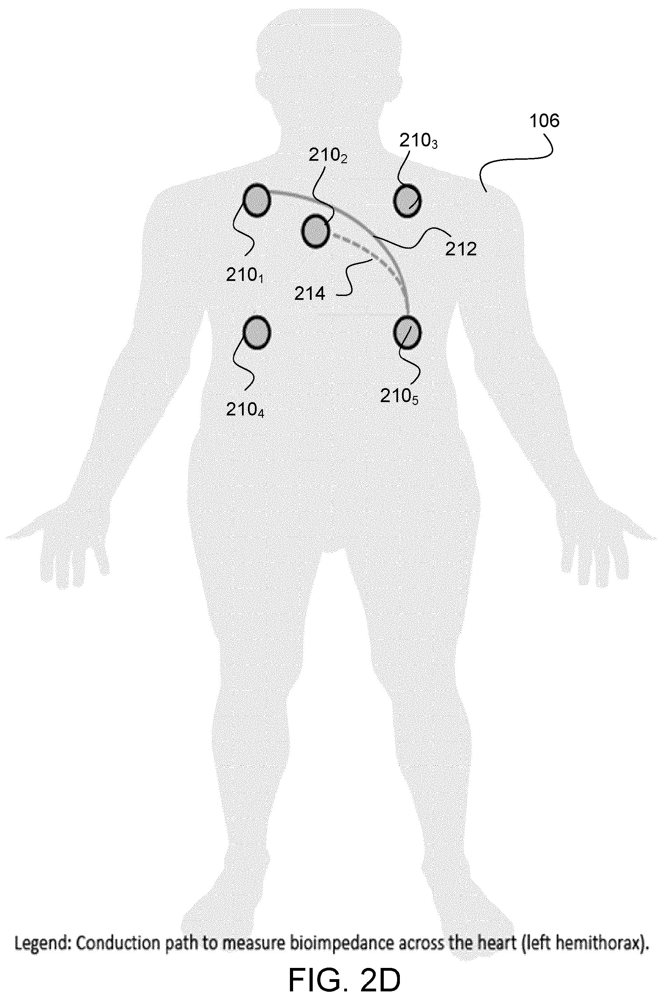

In some embodiments, the ECG configuration from which the ECG and BIA data is captured is a standard 5-lead configuration. With reference to Fig. 2D, an example is illustrated for the 5-lead configuration, whereby five electrode positions 210₁ - 210₅ are located on the chest of the patient 106. In this example, current is injected across a pair of electrodes at positions 200, and 200₅ to create conduction path 212. Active voltage measurements are taken across electrodes located at electrode positions 210₂ and 210₅ along voltage measurement path 214. Passive voltage measurements may be obtained from any one of the five electrode positions 210₁ - 210₅.

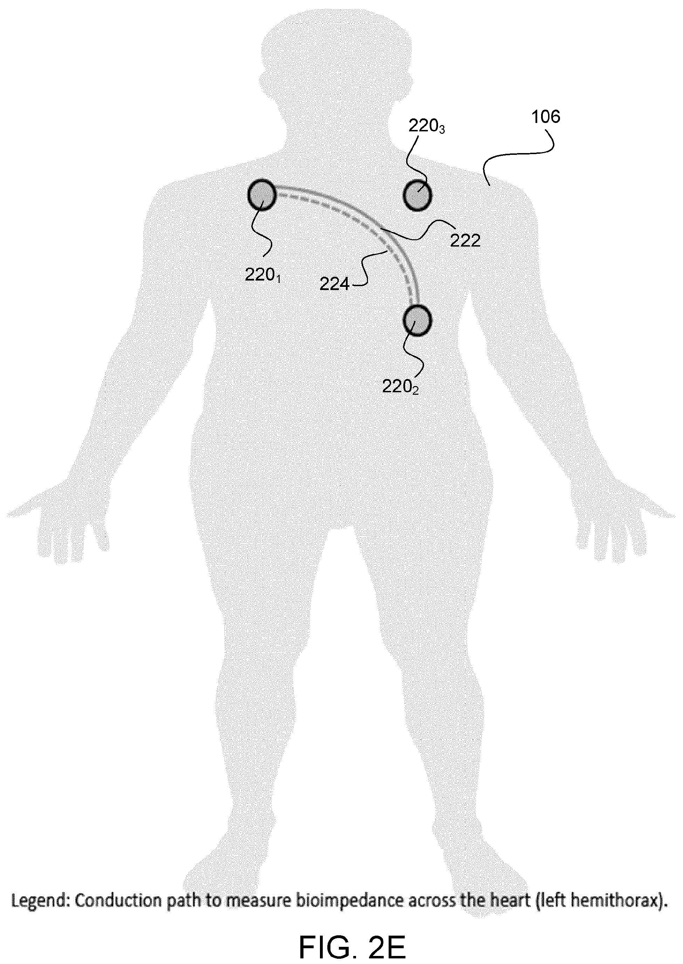

In some embodiments, the ECG configuration from which the ECG and BIA data is captured is a standard 3-lead configuration. With reference to Fig. 2E, an example is illustrated for the standard 3-lead configuration, whereby three electrode positions 220₁ - 220₃ are located on the chest of the patient 106. In this example, current is injected across a pair of electrodes at positions 220₁ and 220₂ to create conduction path 222. Active voltage measurements are taken across electrodes located at electrode positions 220, and 220₂ along voltage measurement path 224. Passive voltage measurements may be obtained from any one of the three electrode positions 220₁ - 220₃.

It will be understood that the current injection and voltage measurement positions illustrated in Figs. 2B-2E are exemplary only and BIA data may be obtained using any pairs of electrodes that lie within a conduction path. It will also be understood that other ECG configurations may also be used, by providing additional electrodes to a standard configuration or by providing a non-standard or alternative configuration.

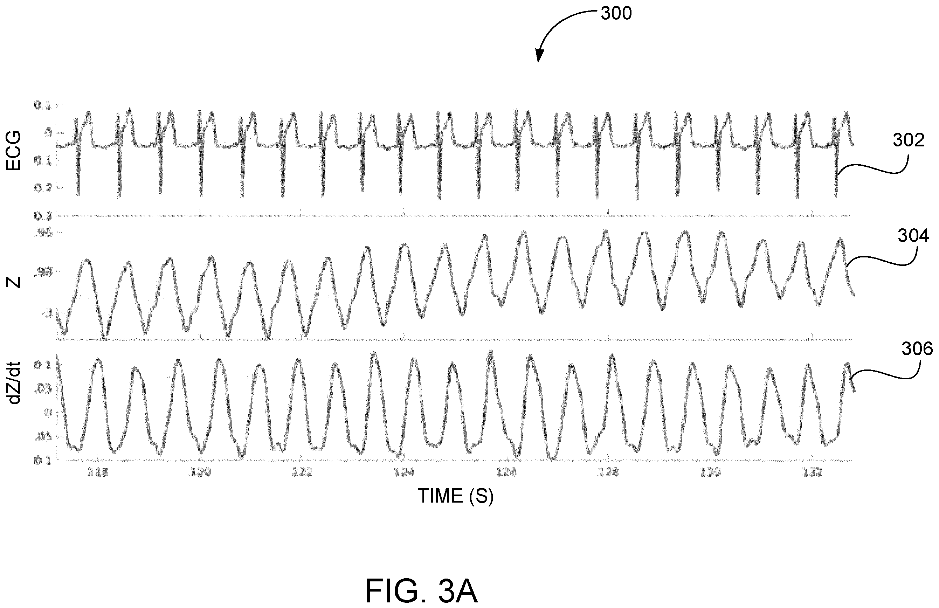

The monitoring device 102 is configured for displaying at least one output based on the BIA measurements, the ECG measurements, or a combination thereof. With reference to Fig. 3A, there is illustrated an example output 300. An example ECG tracing 302 represents the heart's electrical activity as voltages over time. An example bioimpedance readout includes impedance over time (Z) 304 and its derivative (dZ/dt) 306 to show the resistance level of the body's tissue against the current injected therein. In some embodiments, multi-frequency BIA (MF-BIA) is performed, whereby at least two different frequencies of alternating current are injected and active voltage measurements are performed. In some embodiments, bioimpedance spectroscopy (BIS) is performed, whereby impedance is measured at a large number of different frequencies (e.g. 256 frequencies from 3 kHz to 1000 kHz) of alternating current.

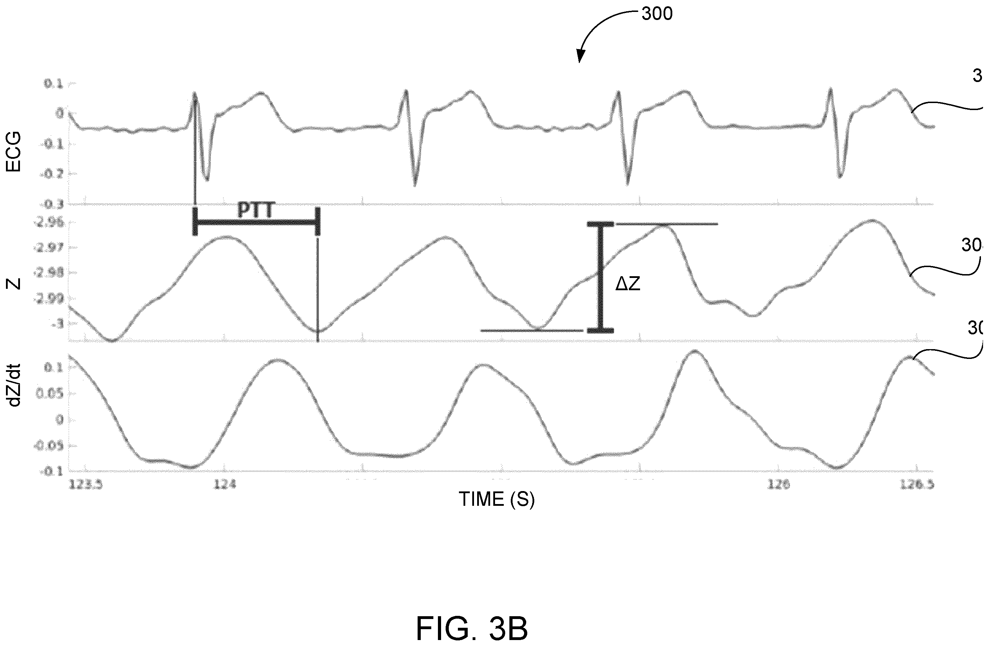

In some embodiments, the output 300 comprises impedance plethysmography (IPG) readouts representative of cardiovascular function over time. IPG readouts may be presented as a function of changes in bioimpedance waveform amplitude over time or bioimpedance waveform timing based on a combination of the ECG data and the BIA data. Measurements for IPG are acquired through electrodes positioned on a body of the patient in an ECG configuration rather than an IPG configuration, whereby the IPG configuration comprises additional electrodes placed on a body of the patient (e.g. on a neck, abdomen, or other parts of a limb) that are not part of an ECG configuration. As shown in Fig. 3B, IPG measures may be computed from changes in bioimpedance amplitude (e.g. ΔZ) and bioimpedance timing relative to the ECG tracing 302 (e.g. Pulse Transit Time (PTT)) obtained from electrodes at specific electrode locations on the body. The IPG measures may be used to derive surrogates of cardiovascular parameters.

IPG results are computed by waveform analysis of a time-series of BIA measurements superposed with a time-series of ECG measurements. Each BIA measurement stems from an activated set of current-injecting electrodes and voltage-measuring electrodes, which is spatially mapped to a corresponding distribution of anatomical structures based on an atlas (i.e. a collection of maps) of conduction paths that is programmed in the system. The atlas of conduction paths is specific for a given configuration and activation of electrodes (i.e. measurement pattern). The system comprises a custom atlas of conduction paths designed specifically for an ECG configuration of electrodes and for an activation of electrodes spatially mapped to capture the cardiac chambers, great vessels, and peripheral vessels. The custom atlas comprises conduction paths for different genders and body sizes, which have been developed by superimposing an ECG configuration of electrodes on 3-dimensional radiographic models of a body and simulating the conduction paths produced by activated sets of current-injecting electrodes and voltage-measuring electrodes.

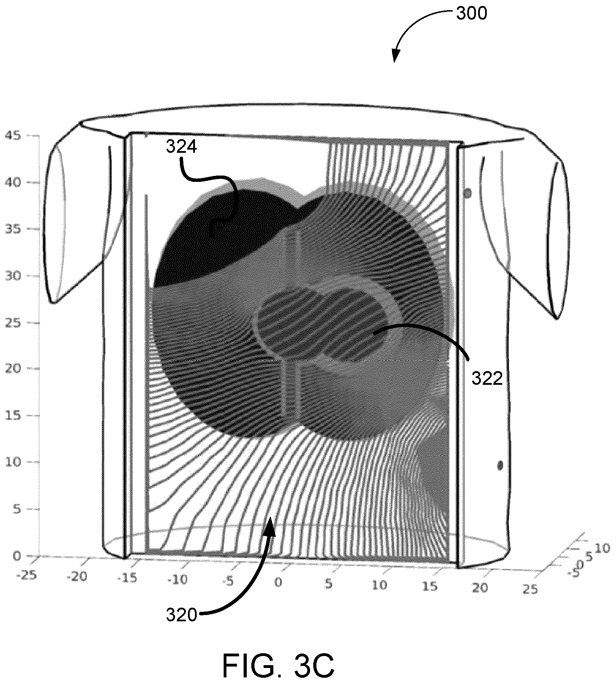

In some embodiments, the output 300 comprises impedance tomography (ITG) readouts representative of cardiovascular and pulmonary function over time. ITG readouts may be presented as static or dynamic images of a heart or a lung of the patient based on a combination of the ECG measurements and the BIA measurements. Measurements for ITG are acquired through electrodes positioned on a body of the patient in an ECG configuration rather than an ITG configuration, whereby the ITG configuration comprises additional electrodes placed on a body of the patient (e.g. spanning the circumference of a torso or the length of a limb) that are not part of an ECG configuration. An example output 300 comprising ITG readouts is illustrated in Fig. 3C. A spatial distribution 320 of voltage potentials having sensitivity to local changes in conductivity caused by flowing air or blood are recorded over time by voltage measurements through the precordial ECG electrodes in order to create an image reconstruction of the cardiovascular 322 and pulmonary 324 anatomy and function.

ITG images are constructed by back-projection of a vector of voltage measurements onto a volumetric image of a body. Each BIA measurement stems from an activated set of current-injecting electrodes and voltage-measuring electrodes, which is spatially mapped to a corresponding distribution of anatomical structures based on an atlas (i.e. collection of maps) of sensitivity matrices (i.e. maps of activated voltage potentials' anatomical landmarks) that is programmed in the system. The atlas of sensitivity matrices is specific for a given configuration and activation of electrodes. The system comprises a custom atlas of sensitivity matrices designed specifically for an ECG configuration of electrodes and for an activation of electrodes spatially mapped to capture the cardiac chambers, great vessels, and lungs. The custom atlas comprises sensitivity matrices for different genders and body sizes, which have been developed by superimposing an ECG configuration of electrodes on 3-dimensional radiographic models of a body and simulating the voltage potentials produced by activated sets of current-injecting electrodes and voltage-measuring electrodes. Lastly, ITG images are refined by filtering to reduce image blurring without significantly increasing image noise. The system comprises a custom pipeline of tuneable filters designed specifically for images constructed using an ECG configuration of electrodes.

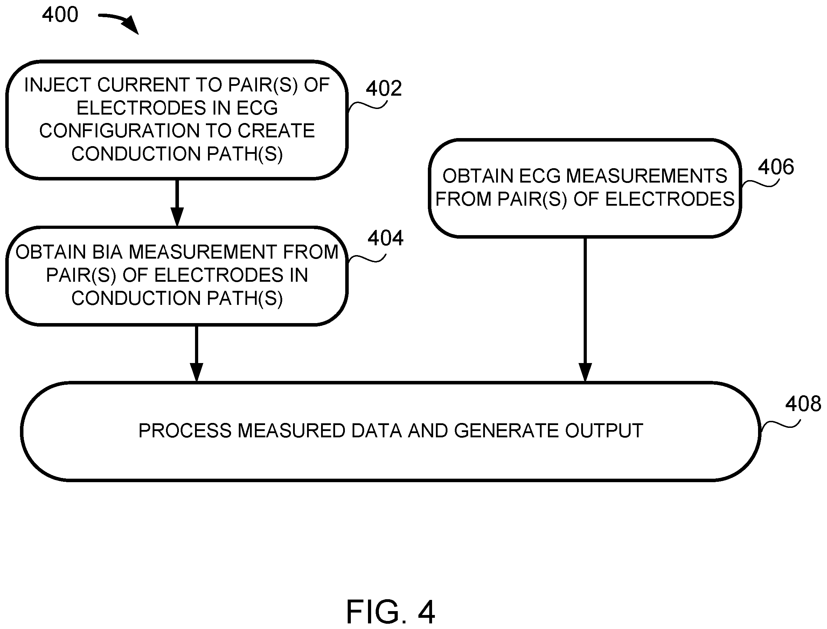

With reference to Fig. 4, there is illustrated an example method 400 for assessing cardiovascular function of a patient, for example using the medical system 100 as illustrated in any one of Figs. 1A-1D. At step 402, alternating current is injected across at least one first pair of electrodes selected from a plurality of electrodes in contact with the body of the patient. The plurality of electrodes are positioned in an ECG configuration, for example a 12-lead configuration, a 5-lead configuration, a 3-lead configuration, and the like. Injection of the current creates at least one conduction path across the first pair of electrodes. In some embodiments, multi-frequency current is applied. At step 404, BIA measurements are obtained from at least one second pair of electrodes that lie within the conduction path created by injecting the current into the first pair of electrodes. In some embodiments, the first pair of electrodes and the second pair of electrodes are the same electrodes. That is to say, the current is injected and the voltage is measured across the same pair of electrodes. In some embodiments, the first pair of electrodes and the second pair of electrodes have one electrode in common, i.e. the current is injected across the first pair of electrodes and the voltage is measured across a third electrode and one of the electrodes forming the first pair of electrodes. In some embodiments, the first pair of electrodes and the second pair of electrodes are independent.

At step 406, ECG measurements are obtained from at least one third pair of electrodes. This may be done concurrently with steps 402, 404. Step 406 may also be performed before or after steps 402, 404. In some embodiments, steps 402, 404, 406 are performed concurrently, in accordance with a measurement pattern that ensures that ECG measurements and BIA measurements do not interfere with each other. In some embodiments, steps 402, 404, 406 are performed concurrently and filtering techniques are used to isolate ECG data from BIA data. In some embodiments, the third pair of electrodes has one or both electrodes in common with the second pair of electrodes and/or the first pair of electrodes.

In some embodiments, steps 402 and 404 of the method 400 are inhibited when a cardiac implanted electronic device is detected in the body of the patient. For example, an additional step may be performed prior to beginning the test to ensure the absence of such a device.

At step 408, measured data is processed and at least one output based on the BIA data, the ECG data, or a combination thereof is generated. The output comprises surrogates for parameters of cardiovascular function that are predicted from the ECG and BIA data. The surrogates are predicted using deep learning algorithms, and are outputted in a clinical format. The output may comprise standard ECG readouts representative of cardiovascular function. The output may comprise BIA readouts of impedance and phase angle representative of cardiovascular function and body composition. The output may comprise impedance plethysmography readouts representative of cardiovascular function over time. The output may comprise impedance tomography readouts representative of cardiovascular and pulmonary function over time. The output may comprise static or dynamic images of a heart of the patient based on the ECG measurements and the BIA measurements.

In some embodiments, processing the measured data at step 408 comprises detecting the likelihood of heart failure diagnosis and its severity, whereby severity may be indicative of prognostic risk and therapeutic response, by using deep learning algorithms to analyze multi-frequency BIA signals with multichannel ECG signals to predict parameters of cardiovascular function and hemodynamics. For example, the method 400 outputs a predicted level or proxy of brain natiuretic peptide (BNP) or N-terminal brain natiuretic peptide (NT-proBNP) to represent a diagnosis of heart failure and its current severity, whereby severity is indicative of prognosis and response to treatment. Alternatively or in combination therewith, the method 400 outputs a predicted level or proxy of central venous pressure (CVP) similar to right atrial pressure (RAP) to represent a diagnosis of heart failure and its current severity, whereby severity is indicative of prognosis and response to treatment. Alternatively or in combination therewith, the method 400 outputs a predicted level or proxy of pulmonary capillary wedge pressure (ePCWP) similar to left atrial pressure (LAP) to represent a diagnosis of heart failure and its current severity, whereby severity is indicative of prognosis and response to treatment. Alternatively or in combination therewith, the method 400 outputs a predicted level or proxy of ventricular ejection fraction (VEF) similar to myocardial performance to represent the diagnosis of heart failure and its current severity, whereby severity is indicative of prognosis and response to treatment. Alternatively or in combination therewith, the method 400 outputs the predicted level or proxy of late gadolinium enhancement (LGE) similar to myocardial viability to represent the etiology of heart failure and its severity, whereby severity is indicative of prognosis and response to treatment. Alternatively or in combination therewith, processing the measured data at step 408 comprises detecting the likelihood of pulmonary edema such as resulting from reduction of effective blood transit through a heart (e.g. heart failure). Alternatively or in combination therewith, processing the measured data at step 408 comprises detecting the likelihood of peripheral edema such as globally resulting from reduction of blood leaving a heart (e.g. heart failure) or segmentally resulting from reduction of blood leaving a limb (e.g. deep vein thrombosis). Alternatively or in combination therewith, processing the measured data at step 408 comprises detecting the likelihood of peripheral arterial disease such as resulting from reduction of blood supply to a limb (e.g. limb ischemia). Alternatively or in combination therewith, processing the measured data at step 408 comprises detecting the likelihood of heart injury such as resulting from reduction of blood supply to a heart (e.g. myocardial ischemia) or from effects of toxins to a heart (e.g. cancer therapy). Alternatively or in combination therewith, processing the measured data at step 408 comprises detecting the likelihood of heart viability such as resulting from sustained lack of blood supply to a heart (e.g. myocardial infarction).

In some embodiments, the method 400 facilitates a user's interpretation by reporting heart failure results in the format of electronically-derived surrogates of familiar clinical parameters. For example, the method 400 outputs the predicted level or proxy of brain natiuretic peptide (BNP) or N-terminal brain natiuretic peptide (NT-proBNP) as the "eBNP" or "eNT-proBNP", respectively, where the prefix "e" denotes the electronically-derived version. Alternatively or in combination therewith, the method 400 outputs the predicted level or proxy of central venous pressure (CVP) or right atrial pressure (RAP) as the "eCVP" or "eRAP", respectively. Alternatively or in combination therewith, the method 400 outputs the predicted level or proxy of pulmonary capillary wedge pressure (PCWP) or left atrial pressure (LAP) as the "ePCWP" or "eLAP", respectively. Alternatively or in combination therewith, the method 400 outputs the predicted level or proxy of left ventricular ejection fraction (LVEF) or right ventricular ejection fraction (RVEF) as the "eLVEF" or "eRVEF", respectively. Alternatively or in combination therewith, the method outputs the predicted distribution of late gadolinium enhancement (LGE) as the "eLGE". Alternatively or in combination therewith, the method outputs the predicted distribution of ankle-brachial index (ABI) as the "eABI".

In some embodiments, the method 400 supports pharmacologic treatment decisions and monitors treatment effects, and processing the measured data at step 408 comprises using deep learning algorithms to analyze multi-frequency BIA signals with multichannel ECG signals to model the changes in parameters of cardiovascular function and hemodynamics. For example, the method 400 outputs suggestions for administration and tailored dosage of intravenous fluids, with the anticipated hemodynamic responsiveness of a given individual. Alternatively or in combination therewith, the method 400 outputs suggestions for administration and tailored dosage of diuretic drugs, which may include starting, stopping, increasing or decreasing these drugs. Alternatively or in combination therewith, the method 400 outputs suggestions for administration of inotropic therapy.

In some embodiments, the method 400 supports pharmacologic treatment decisions and monitors treatment effects, and processing the measured data at step 408 comprises using deep learning algorithms to analyze multi-frequency BIA signals with multichannel ECG signals to model the changes in parameters of body composition and pharmacokinetics. For example, the method 400 outputs the predicted level or proxy of hydrophilic drug concentrations in the body and suggestions for tailored dosage of these drugs, which may include off-label dosages to achieve ideal concentrations of these drugs in a particular person. Alternatively or in combination therewith, the aforementioned drugs would comprise anticoagulant drugs, wherein tailored dosage could reduce the risk of bleeding complications. Alternatively or in combination therewith, the aforementioned drugs would comprise chemotherapy drugs, wherein tailored dosage could reduce the risk of toxicity effects.

In some embodiments, processing the measured data at step 408 comprises detecting the likelihood of future adverse health events by using deep learning algorithms to integrate the heart failure and frailty readouts to predict patient-level risk. For example, the method 400 outputs the predicted probability of death, heart failure related decompensation, readmission, or other adverse health events. Alternatively or in combination therewith, the method 400 outputs a measure of cardio-geriatric risk that reflects cumulative cardiac and geriatric impairments. Alternatively or in combination therewith, the method 400 outputs suggestions for optimization of care to therapeutically target the risk features identified.

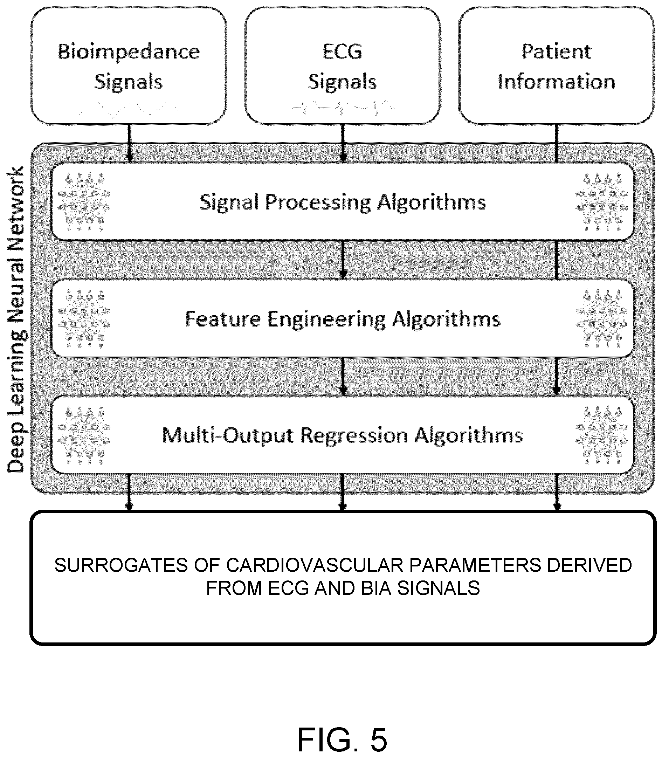

With reference to Fig. 5, there is illustrated schematically an example analytical pipeline for predicting parameters of cardiovascular function from the ECG and BIA data using deep learning algorithms, and outputting the predicted parameters in a clinical format. An input layer consists of (i) BIA signals acquired at low, mid, and high current injection frequencies between specified pairs of electrodes within the standard ECG configuration; (ii) ECG signals acquired concomitantly with the BIA signals from the same electrodes, and (iii) patient information such as age, sex, height, and weight. The middle layers consist of deep learning algorithms for signal processing, feature extraction and engineering, classification and regression. The structure of the deep learning model is an ensemble of deep neural networks for signal time series classification and regression, including bilateral long-short-term memory (LSTM) recurrent neural networks. The output consists of surrogates of cardiovascular parameters derived from the ECG and BIA data. For example, the surrogates of cardiovascular parameters may comprise surrogates of natriuretic peptide levels, cardiac or vascular pressures, cardiac ejection fraction, and vascular stiffness. Clinical parameters such as likelihood and severity of heart failure, ideal dosage of diuretic or anticoagulant drugs, rating of frailty, risk of mortality or morbidity, readiness for hospital discharge or need for hospital admission may also be derived and/or predicted from the ECG and BIA data. Body composition parameters such as muscle and fat mass, intra and extra cellular water, and volume distribution may also form part of the output.

The model is trained with the ECG and BIA signals concomitantly acquired from a given ECG electrode configuration with minimal interference between signals and maximal fidelity (achieved by tuning the measurement sequence and signal filters), as if these signals had been independently acquired from a dedicated device with the optimal complete electrode configuration for that purpose. Ensuring minimal interference and maximal fidelity provides robust training signals to the model, and the concomitant acquisition allows the model to analyze the temporal relationships between beat-to-beat BIA and ECG signal features. Traditionally, the standard ECG configuration is inherently suboptimal for the purpose of BIA due to the confined number and positioning of electrodes designed for the purpose of ECG. This standard ECG electrode configuration is especially suboptimal for advanced BIA functionalities such as impedance plethysmography and impedance tomography.

The traditional output data of BIA is presented primarily in terms of impedance values and phase angle values for different body regions, and secondarily in terms of estimated body composition parameters (lean mass, fat mass, body water) based on these raw values and user-entered data. These estimates of body composition parameters are based on rudimentary regression equations, which are known to be inaccurate. The traditional output of ECG is presented primarily in terms of graphical ECG tracings and secondarily in terms of computer-assisted interpretations of these tracings for certain cardiac anomalies (atrial anomaly, ventricular hypertrophy, ventricular ischemia, metabolic disturbance, conduction disturbance, and arrhythmia). These ascertainments of cardiac anomalies are often inaccurate and limited in scope. The traditional output of BIA and ECG data is presented primarily in terms of graphical BIA and ECG tracings with intervals of time measured between the tracings, and secondarily in terms of their individual outputs listed above. In contrast, the analytical pipeline of Fig. 5 generates output by post-processing and analyzing the BIA and ECG data and providing surrogates of cardiovascular parameters that are traditionally derived from imaging, blood tests, pressures, clinical characteristics, and the like. These surrogates predict the cardiovascular parameters of interest, and may be scaled and/or calibrated to be presented in a clinical format. The clinical format is understood to refer to clinical, biochemical, or radiographic markers which are already familiar for clinicians and actionable based on similar cut-offs. This is accomplished by adding successive layers to the deep learning model that first filter the raw signals, then extract the relevant features, then predict the traditional outputs, and finally generate the surrogate outputs to be presented in a test report.

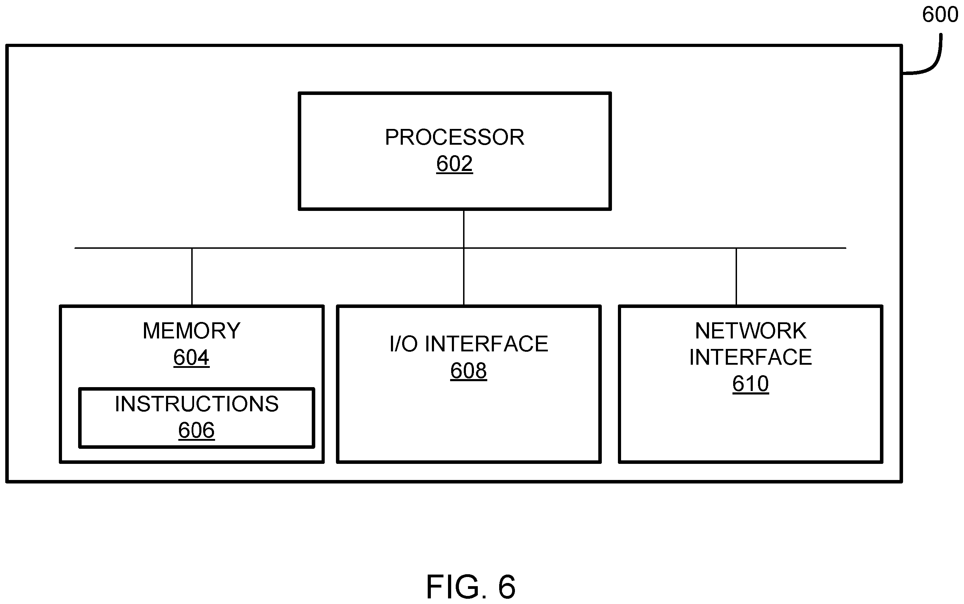

The method 400 described herein may be implemented in a combination of both hardware and software. These embodiments may be implemented on programmable computers, each computer including at least one processor, a data storage system (including volatile memory or non-volatile memory or other data storage elements or a combination thereof), and at least one communication interface. Fig. 6 is a schematic diagram of a computing device 600, exemplary of the monitoring device 102. As depicted, computing device 600 includes at least one processor 602, a memory 604 having program instructions 606 stored thereon, at least one I/O interface 608, and at least one network interface 610.

Each processor 602 may be, for example, any type of general-purpose microprocessor or microcontroller, a digital signal processing (DSP) processor, an integrated circuit, a field programmable gate array (FPGA), a reconfigurable processor, a programmable read-only memory (PROM), or any combination thereof.

Memory 604 may include a suitable combination of any type of computer memory that is located either internally or externally such as, for example, random-access memory (RAM), read-only memory (ROM), compact disc read-only memory (CDROM), electro-optical memory, magneto-optical memory, erasable programmable read-only memory (EPROM), and electrically-erasable programmable read-only memory (EEPROM), Ferroelectric RAM (FRAM) or the like. Program instructions 606 are applied to input data to perform the functions described herein and to generate output information. The output information is applied to one or more output devices.

Each I/O interface 608 enables computing device 600 to interconnect with one or more input devices, such as a keyboard, mouse, camera, touch screen and a microphone, or with one or more output devices such as a display screen and a speaker.

Each network interface 610 enables computing device 600 to communicate with other components, to exchange data with other components, to access and connect to network resources, to serve applications, and perform other computing applications by connecting to a network (or multiple networks) capable of carrying data including the Internet, Ethernet, plain old telephone service (POTS) line, public switch telephone network (PSTN), integrated services digital network (ISDN), digital subscriber line (DSL), coaxial cable, fiber optics, satellite, mobile, wireless (e.g. Wi-Fi, WiMAX), SS7 signaling network, fixed line, local area network, wide area network, and others, including any combination of these.

For simplicity only one computing device 600 is shown but the monitoring system 100 may include more computing devices 600 operable by users to access remote network resources and exchange data. The computing devices 600 may be the same or different types of devices. The computing device components may be connected in various ways including directly coupled, indirectly coupled via a network, and distributed over a wide geographic area and connected via a network (which may be referred to as "cloud computing"). The term "connected" or "coupled to" may include both direct coupling (in which two elements that are coupled to each other contact each other) and indirect coupling (in which at least one additional element is located between the two elements).

For example, and without limitation, the computing device 600 may be a server, network appliance, embedded device, computer expansion module, personal computer, laptop, personal data assistant, cellular telephone, smartphone device, tablet, or any other computing device capable of being configured to carry out part or all of the method 400 described herein.

The technical solution of embodiments may be in the form of a software product. The software product may be stored in a non-volatile or non-transitory storage medium, which can be a compact disk read-only memory (CD-ROM), a USB flash disk, or a removable hard disk. The software product includes a number of instructions that enable a computer device (personal computer, server, or network device) to execute the methods provided by the embodiments.

The embodiments described herein are implemented by physical computer hardware, including computing devices, servers, receivers, transmitters, processors, memory, displays, and networks. The embodiments described herein provide useful physical machines and particularly configured computer hardware arrangements. The embodiments described herein are directed to electronic machines and methods implemented by electronic machines adapted for processing and transforming electromagnetic signals which represent various types of information. The embodiments described herein pervasively and integrally relate to machines, and their uses; and the embodiments described herein have no meaning or practical applicability outside their use with computer hardware, machines, and various hardware components. Substituting the physical hardware particularly configured to implement various acts for non-physical hardware, using mental steps for example, may substantially affect the way the embodiments work. Such computer hardware limitations are clearly essential elements of the embodiments described herein, and they cannot be omitted or substituted for mental means without having a material effect on the operation and structure of the embodiments described herein. The computer hardware is essential to implement the various embodiments described herein and is not merely used to perform steps expeditiously and in an efficient manner.

Although the embodiments have been described in detail, it should be understood that various changes, substitutions and alterations can be made herein without departing from the scope as defined by the appended claims.

Moreover, the scope of the present application is not intended to be limited to the particular embodiments described in the specification. As one of ordinary skill in the art will readily appreciate from the present disclosure, processes, machines, manufacture, compositions of matter, means, methods, or steps, presently existing or later to be developed, that perform substantially the same function or achieve substantially the same result as the corresponding embodiments described herein may be utilized. Accordingly, the appended claims are intended to include within their scope such processes, machines, manufacture, compositions of matter, means, methods, or steps.

## Claims

1. A system for assessing cardiovascular functions of a patient (106), the system comprising:
a processor; and
a non-transitory computer-readable medium having stored thereon program instructions executable by the processor for:
acquiring electrocardiogram (ECG) data and bioelectrical impedance analysis (BIA) data concomitantly through a plurality of electrodes (104) positioned in an ECG configuration, the ECG data comprising a time-series of ECG measurements and the BIA data comprising a time-series of BIA measurements superposed with the time-series of ECG measurements, each BIA measurement stemming from an activated set of the plurality of electrodes (104) that is spatially mapped to a distribution of anatomical structures based on an atlas of conduction paths specific for the ECG configuration and activation of the plurality of electrodes (104);
predicting surrogates of parameters of cardiovascular function from the ECG data and the BIA data using a trained deep learning algorithm, the parameters of cardiovascular function being cardiovascular parameters of interest traditionally derived from at least one of imaging, blood tests, and pressure measurements; and
outputting the surrogates of the parameters of cardiovascular function in a clinical format.

2. The system of claim 1, wherein predicting the surrogates of the parameters of cardiovascular function comprises generating impedance plethysmography (IPG) data representative of cardiovascular function over time from the ECG data and BIA data.

3. The system of claims 1 or 2, wherein predicting the surrogates of the parameters of cardiovascular function comprises generating impedance tomography (ITG) data, comprising static or dynamic images of a heart and a lung of the patient (106), representative of cardiovascular and pulmonary function over time from the ECG data and BIA data.

4. The system of any one of claims 1 to 3, wherein predicting the surrogates of the parameters of cardiovascular function comprises predicting a level or proxy of at least one of the parameters of cardiovascular function to represent a likelihood and associated severity of a diagnosis of at least one of heart failure, pulmonary edema, peripheral edema, peripheral arterial occlusion, and peripheral venous occlusion, whereby severity informs a prognosis and treatment of the patient (106).

5. The system of any one of claims 1 to 4, wherein predicting the surrogates of the parameters of cardiovascular function comprises predicting a level or proxy of brain natiuretic peptide (BNP) or N-terminal brain natiuretic peptide (NT-proBNP) to represent a likelihood and associated severity of a diagnosis of heart failure, whereby severity informs a prognosis and treatment of the patient (106).

6. The system of any one of claims 1 to 5, wherein the program instructions are further executable by the processor for:
modeling changes in the parameters of cardiovascular function to support pharmacologic treatment decisions and monitor treatment effects; and
presenting, based on the modeling of the changes in the parameters of cardiovascular function, one or more suggestions for administration and tailored dosage of one of diuretic drugs and intravenous fluids in accordance with an anticipated hemodynamics responsiveness.

7. The system of any one of claims 1 to 6, wherein the program instructions are further executable by the processor for:
modeling changes in parameters of body composition to support pharmacologic treatment decisions and monitor treatment effects; and
predicting, based on the modeling of the changes in the parameters of body composition, a level or proxy of hydrophilic drug concentrations in a body of the patient (106) and suggestions for tailored dosage of hydrophilic drugs, the hydrophilic drugs comprising anticoagulant drugs and/or chemotherapy drugs.

8. The system of any one of claims 1 to 7, wherein the program instructions are further executable by the processor for applying the deep learning algorithms for detecting a likelihood of cardiovascular disease or cardiotoxicity and an associated severity,
preferably wherein the program instructions are further executable by the processor for applying the deep learning algorithms for detecting a likelihood of frailty and an associated severity, and predicting a likelihood of ancillary comorbid diagnoses having a correlation with a likelihood of frailty, and
preferably wherein the program instructions are further executable by the processor for predicting a likelihood of future adverse health events using at least one of the likelihood of cardiovascular disease and the likelihood of frailty, and for predicting a probability of death, decompensation, readmission, or other adverse health events.

9. The system of any one of claims 1 to 8, wherein the program instructions are further executable by the processor for predicting a readiness for hospital discharge or a need for hospital admission.

10. A method for assessing cardiovascular functions of a patient (106), the method comprising, at a processor:
inputting ECG data and BIA data acquired concomitantly through a plurality of electrodes (104) positioned in an ECG configuration, to an input layer of a deep learning model, where the ECG data comprise a time-series of ECG measurements and the BIA data comprise a time-series of BIA measurements superposed with the time-series of ECG measurements, each BIA measurement stemming from an activated set of the plurality of electrodes (104) that is spatially mapped to a distribution of anatomical structures based on an atlas of conduction paths specific for the ECG configuration and activation of the plurality of electrodes (104);
predicting surrogates of parameters of cardiovascular function from the ECG data and the BIA data using a trained deep learning algorithm, the parameters of cardiovascular function being cardiovascular parameters of interest traditionally derived from at least one of imaging, blood tests, and pressure measurements; and
outputting the surrogates of the parameters of cardiovascular function in a clinical format.

11. The method of claim 10, wherein predicting the surrogates of the parameters of cardiovascular function comprises generating impedance plethysmography (IPG) data representative of cardiovascular function over time from the ECG data and BIA data.

12. The method of claims 10 or 11, wherein predicting the surrogates of the parameters of cardiovascular function comprises generating impedance tomography (ITG) data, including static or dynamic images of a heart and a lung of the patient (106), representative of cardiovascular and pulmonary function over time from the ECG data and BIA data.

13. The method of any one of claims 10 to 12, wherein predicting the surrogates of the parameters of cardiovascular function comprises predicting a level or proxy of at least one of the parameters of cardiovascular function.

14. The method of any one of claims 10 to 13, wherein predicting the surrogates of the parameters of cardiovascular function comprises predicting a level or proxy of brain natiuretic peptide (BNP) or N-terminal brain natiuretic peptide (NT-proBNP).

15. The method of any one of claims 10 to 14, further comprising:
modeling changes in the parameters of cardiovascular function and hemodynamics.

16. The method of any one of claims 10 to 15, further comprising:
modeling changes in parameters of body composition; and
predicting, based on the modeling of the changes in the parameters of body composition, a level or proxy of hydrophilic drug concentrations in a body of the patient (106).

## Patentansprüche

1. System zur Beurteilung der kardiovaskulären Funktionen eines Patienten (106), wobei das System umfasst:
einen Prozessor; und
ein nichtflüchtiges, computerlesbares Medium, das darauf gespeicherte Programmanweisungen aufweist, die von dem Prozessor für Folgendes ausführbar sind:
gleichzeitiges Erfassen von Elektrokardiogramm-(EKG-)Daten und bioelektrischer Impedanzanalyse-(BIA-)Daten über eine Vielzahl von in einer EKG-Konfiguration positionierten Elektroden (104), wobei die EKG-Daten eine Zeitreihe von EKG-Messungen umfassen und die BIA-Daten eine Zeitreihe von BIA-Messungen umfassen, die mit der Zeitreihe von EKG-Messungen überlagert sind, wobei jede BIA-Messung aus einem aktivierten Satz der Vielzahl von Elektroden (104) stammt, der räumlich einer Verteilung anatomischer Strukturen zugeordnet ist, auf der Grundlage eines Atlasses von Leitungswegen, der spezifisch für die EKG-Konfiguration und Aktivierung der Vielzahl von Elektroden (104) ist;
Vorhersagen von Surrogaten für Parameter der kardiovaskulären Funktion aus den EKG-Daten und den BIA-Daten mittels eines trainierten Deep-Learning-Algorithmus, wobei die Parameter der kardiovaskulären Funktion kardiovaskuläre Parameter von Interesse sind, die traditionell aus mindestens einer von Bildgebung, Bluttests und Druckmessungen abgeleitet werden; und
Ausgeben der Surrogate der Parameter der kardiovaskulären Funktion in einem klinischen Format.

2. System nach Anspruch 1, wobei das Vorhersagen der Surrogate der Parameter der kardiovaskulären Funktion das Erzeugen von Impedanz-Plethysmographie-(IPG-)Daten umfasst, die die kardiovaskuläre Funktion über die Zeit aus den EKG-Daten und BIA-Daten darstellen.

3. System nach Ansprüchen 1 oder 2, wobei das Vorhersagen der Surrogate der Parameter der kardiovaskulären Funktion das Erzeugen von Impedanztomographie(ITG)-Daten umfasst, die statische oder dynamische Bilder des Herzens und der Lunge des Patienten (106) umfassen, die die kardiovaskuläre und pulmonale Funktion über die Zeit aus den ECG-Daten und BIA-Daten darstellen.

4. System nach einem der Ansprüche 1 bis 3, wobei das Vorhersagen der Surrogate der Parameter der kardiovaskulären Funktion das Vorhersagen eines Niveaus oder Proxys von mindestens einem der Parameter der kardiovaskulären Funktion umfasst, um die Wahrscheinlichkeit und den damit verbundenen Schweregrad einer Diagnose von mindestens einem von Herzinsuffizienz, Lungenödem, peripherem Ödem, peripherer arterieller Okklusion und peripherer Venenokklusion darzustellen, wobei der Schweregrad einer Prognose und Behandlung des Patienten (106) zugrundeliegt.

5. System nach einem der Ansprüche 1 bis 4, wobei das Vorhersagen der Surrogate der Parameter der kardiovaskulären Funktion das Vorhersagen eines Niveaus oder Proxys des Brain Natriuretic Peptides (BNP) oder N-terminalen Brain Natriuretic Peptides (NT-proBNP) umfasst, um die Wahrscheinlichkeit und den damit verbundenen Schweregrad einer Diagnose von Herzinsuffizienz darzustellen, wobei der Schweregrad einer Prognose und Behandlung des Patienten (106) zugrundeliegt.

6. System nach einem der Ansprüche 1 bis 5, wobei die Programmanweisungen weiter von dem Prozessor ausführbar sind zum:
Modellieren von Veränderungen der Parameter der kardiovaskulären Funktion zur Unterstützung pharmakologischer Behandlungsentscheidungen und zur Überwachung der Behandlungseffekte; und
Aufzeigen, auf der Grundlage des Modellierens der Veränderungen der Parameter der kardiovaskulären Funktion, eines oder mehrerer Vorschläge für die Verabreichung und individuell angepasste Dosierung von einem von Diuretika und intravenösen Flüssigkeiten in Übereinstimmung mit einer erwarteten hämodynamischen Reaktionsfähigkeit.

7. System nach einem der Ansprüche 1 bis 6, wobei die Programmanweisungen weiter von dem Prozessor ausführbar sind zum:
Modellieren von Veränderungen der Parameter der Körperzusammensetzung zur Unterstützung pharmakologischer Behandlungsentscheidungen und zur Überwachung der Behandlungseffekte; und
Vorhersagen, auf der Grundlage des Modellierens der Veränderungen der Parameter der Körperzusammensetzung, eines Niveaus oder Proxys der Konzentrationen hydrophiler Arzneimittel in dem Körper des Patienten (106) sowie Vorschläge für eine individuell angepasste Dosierung hydrophiler Arzneimittel, wobei die hydrophilen Arzneimittel Antikoagulanzien und/oder Chemotherapeutika umfassen.

8. System nach einem der Ansprüche 1 bis 7, wobei die Programmanweisungen weiter von dem Prozessor ausführbar sind, um die Deep-Learning-Algorithmen zur Erkennung einer Wahrscheinlichkeit für Herz-Kreislauf-Erkrankungen oder Kardiotoxizität und einen damit verbundenen Schweregrad anzuwenden,
wobei die Programmanweisungen bevorzugt weiter von dem Prozessor ausführbar sind, um die Deep-Learning-Algorithmen anzuwenden, um eine Wahrscheinlichkeit für Gebrechlichkeit und einen damit verbundenen Schweregrad zu erkennen sowie eine Wahrscheinlichkeit für begleitende komorbide Diagnosen vorherzusagen, die eine Korrelation mit einer Wahrscheinlichkeit für Gebrechlichkeit aufweisen, und
wobei die Programmanweisungen bevorzugt weiter von dem Prozessor ausführbar sind, um eine Wahrscheinlichkeit zukünftiger negativer Gesundheitsereignisse unter Verwendung mindestens einer von der Wahrscheinlichkeit für Herz-Kreislauf-Erkrankungen und Wahrscheinlichkeit für Gebrechlichkeit vorherzusagen und um eine Wahrscheinlichkeit für Tod, Dekompensation, Wiederaufnahme oder andere negative Gesundheitsereignisse vorherzusagen.

9. System nach einem der Ansprüche 1 bis 8, wobei die Programmanweisungen weiter von dem Prozessor ausführbar sind, um eine Bereitschaft zur Entlassung aus dem Krankenhaus oder eine Notwendigkeit einer Aufnahme in das Krankenhaus vorherzusagen.

10. Verfahren zur Beurteilung der kardiovaskulären Funktionen eines Patienten (106), wobei das Verfahren auf einem Prozessor umfasst:
Eingeben von EKG-Daten und BIA-Daten, die gleichzeitig über eine Vielzahl von in einer EKG-Konfiguration positionierten Elektroden (104) erfasst wurden, in eine Eingabeschicht eines Deep-Learning-Modells, wobei die EKG-Daten eine Zeitreihe von EKG-Messungen umfassen und die BIA-Daten eine Zeitreihe von BIA-Messungen umfassen, die mit der Zeitreihe von EKG-Messungen überlagert sind, wobei jede BIA-Messung aus einem aktivierten Satz der Vielzahl von Elektroden (104) stammt, der räumlich einer Verteilung anatomischer Strukturen zugeordnet ist, auf der Grundlage eines Atlasses von Leitungswegen, der spezifisch für die EKG-Konfiguration und Aktivierung der Vielzahl von Elektroden (104) ist;
Vorhersagen von Surrogaten für Parameter der kardiovaskulären Funktion aus den EKG-Daten und den BIA-Daten mittels eines trainierten Deep-Learning-Algorithmus, wobei die Parameter der kardiovaskulären Funktion kardiovaskuläre Parameter von Interesse sind, die traditionell aus mindestens einer von Bildgebung, Bluttests und Druckmessungen abgeleitet werden; und
Ausgeben der Surrogate der Parameter der kardiovaskulären Funktion in einem klinischen Format.

11. Verfahren nach Anspruch 10, wobei das Vorhersagen der Surrogate der Parameter der kardiovaskulären Funktion das Erzeugen von Impedanz-Plethysmographie-(IPG-)Daten umfasst, die die kardiovaskuläre Funktion über die Zeit aus den EKG-Daten und BIA-Daten darstellen.

12. Verfahren nach Ansprüchen 10 oder 11, wobei das Vorhersagen der Surrogate der Parameter der kardiovaskulären Funktion das Erzeugen von Impedanztomographie-(ITG)-Daten umfasst, einschließlich statischer oder dynamischer Bilder des Herzens und der Lunge des Patienten (106), die die kardiovaskuläre und pulmonale Funktion über die Zeit aus den EKG-Daten und BIA-Daten darstellen.

13. Verfahren nach einem der Ansprüche 10 bis 12, wobei das Vorhersagen der Surrogate der Parameter der kardiovaskulären Funktion das Vorhersagen eines Niveaus oder Proxys von mindestens einem der Parameter der kardiovaskulären Funktion umfasst.

14. Verfahren nach einem der Ansprüche 10 bis 13, wobei das Vorhersagen der Surrogate der Parameter der kardiovaskulären Funktion das Vorhersagen eines Niveaus oder Proxys des Brain Natriuretic Peptides (BNP) oder N-terminalen Brain Natriuretic Peptides (NT-proBNP) umfasst.

15. Verfahren nach einem der Ansprüche 10 bis 14, weiter umfassend:
Modellieren von Veränderungen der Parameter der kardiovaskulären Funktion und der Hämodynamik.

16. Verfahren nach einem der Ansprüche 10 bis 15, weiter umfassend:
Modellieren von Veränderungen der Parameter der Körperzusammensetzung; und
Vorhersagen, auf der Grundlage des Modellierens der Veränderungen der Parameter der Körperzusammensetzung, eines Niveaus oder Proxys der Konzentrationen hydrophiler Arzneimittel in dem Körper des Patienten (106).

## Revendications

1. Système d'évaluation des fonctions cardiovasculaires d'un patient (106), le système comprenant :
un processeur ; et
un support non transitoire lisible par ordinateur présentant sur celui-ci des instructions de programme pouvant être exécutées par le processeur pour :
l'acquisition concomitante de données d'électrocardiogramme (ECG) et de données d'analyse d'impédance bioélectrique (BIA) par l'intermédiaire d'une pluralité d'électrodes (104) positionnées dans une configuration d'ECG, les données d'ECG comprenant une série chronologique de mesures d'ECG et les données de BIA comprenant une série chronologique de mesures de BIA superposées aux séries chronologiques de mesures d'ECG, chaque mesure de BIA émanant d'un ensemble activé de la pluralité d'électrodes (104) qui est cartographié spatialement vers une distribution de structures anatomiques sur la base d'un atlas de chemins de conduction spécifiques à la configuration d'ECG et à l'activation de la pluralité d'électrodes (104) ;
la prédiction de substituts de paramètres de fonction cardiovasculaire à partir des données d'ECG et des données de BIA en utilisant un algorithme d'apprentissage profond entraîné, les paramètres de fonction cardiovasculaire étant des paramètres cardiovasculaires d'intérêt traditionnellement dérivés d'au moins l'une parmi l'imagerie, les analyses de sang et les mesures de pression ; et
la délivrance des substituts des paramètres de fonction cardiovasculaire sous un format clinique.

2. Système selon la revendication 1, dans lequel la prédiction des substituts des paramètres de fonction cardiovasculaire comprend la génération de données de pléthysmographie d'impédance (IPG) représentatives de la fonction cardiovasculaire au fil du temps à partir des données d'ECG et des données de BIA.

3. Système selon la revendication 1 ou la revendication 2, dans lequel la prédiction des substituts des paramètres de fonction cardiovasculaire comprend la génération de données de tomographie par impédance (ITG), comprenant des images statiques ou dynamiques d'un cœur et d'un poumon du patient (106), représentatives de la fonction cardiovasculaire et pulmonaire au fil du temps à partir des données d'ECG et des données de BIA.

4. Système selon l'une quelconque des revendications 1 à 3, dans lequel la prédiction des substituts des paramètres de fonction cardiovasculaire comprend la prédiction d'un niveau ou d'un proxy d'au moins l'un des paramètres de fonction cardiovasculaire de telle sorte que celui-ci représente une probabilité et une gravité associée d'un diagnostic d'au moins l'un parmi l'insuffisance cardiaque, l'œdème pulmonaire, l'œdème périphérique, l'occlusion artérielle périphérique et l'occlusion veineuse périphérique, de sorte que la gravité informe sur un pronostic et un traitement du patient (106).

5. Système selon l'une quelconque des revendications 1 à 4, dans lequel la prédiction des substituts des paramètres de fonction cardiovasculaire comprend la prédiction d'un niveau ou d'un proxy de peptide natriurétique cérébral (BNP) ou de peptide natriurétique cérébral N-terminal (NT-proBNP) de telle sorte que celui-ci représente une probabilité et une gravité associée d'un diagnostic d'insuffisance cardiaque, de sorte que la gravité informe sur un pronostic et un traitement du patient (106).

6. Système selon l'une quelconque des revendications 1 à 5, dans lequel les instructions de programme peuvent en outre être exécutées par le processeur pour :
modéliser les modifications des paramètres de la fonction cardiovasculaire pour étayer les décisions de traitement pharmacologique et surveiller les effets du traitement ; et
présenter, sur la base de la modélisation des modifications des paramètres de la fonction cardiovasculaire, une ou plusieurs suggestions d'administration et de dosage personnalisé de l'un des médicaments diurétiques et des fluides intraveineux conformément à une réactivité hémodynamique anticipée.

7. Système selon l'une quelconque des revendications 1 à 6, dans lequel les instructions de programme peuvent en outre être exécutées par le processeur pour :
modéliser les modifications des paramètres de la composition corporelle pour étayer les décisions de traitement pharmacologique et surveiller les effets du traitement ; et
prédire, sur la base de la modélisation des modifications des paramètres de composition corporelle, un niveau ou un proxy de concentrations de médicament hydrophile dans un corps du patient (106) et de suggestions de dosage personnalisé de médicaments hydrophiles, les médicaments hydrophiles comprenant des médicaments anticoagulants et/ou des médicaments chimiothérapeutiques.

8. Système selon l'une quelconque des revendications 1 à 7, dans lequel les instructions de programme peuvent en outre être exécutées par le processeur pour appliquer les algorithmes d'apprentissage profond pour détecter une probabilité de maladie cardiovasculaire ou de cardiotoxicité et une gravité associée,
préférablement dans lequel les instructions de programme peuvent en outre être exécutées par le processeur pour appliquer les algorithmes d'apprentissage profond pour détecter une probabilité de fragilité et une gravité associée, et prédire une probabilité de diagnostics de comorbidités auxiliaires présentant une corrélation avec une probabilité de fragilité, et
préférablement dans lequel les instructions de programme peuvent en outre être exécutées par le processeur pour prédire une probabilité d'événements indésirables de santé futurs en utilisant au moins l'une parmi la probabilité de maladie cardiovasculaire et la probabilité de fragilité, et pour prédire une probabilité de décès, de décompensation, de réadmission ou d'autres événements indésirables de santé.

9. Système selon l'une quelconque des revendications 1 à 8, dans lequel les instructions de programme peuvent en outre être exécutées par le processeur pour prédire une disponibilité pour une décharge à l'hôpital ou un besoin d'admission à l'hôpital.

10. Procédé d'évaluation des fonctions cardiovasculaires d'un patient (106), le procédé comprenant, au niveau d'un processeur :
l'entrée de données d'ECG et de données de BIA acquises de manière concomitante par l'intermédiaire d'une pluralité d'électrodes (104) positionnées dans une configuration d'ECG, dans une couche d'entrée d'un modèle d'apprentissage profond, où les données d'ECG comprennent une série chronologique de mesures d'ECG et les données de BIA comprennent une série chronologique de mesures de BIA superposées aux séries chronologiques de mesures d'ECG, chaque mesure de BIA émanant d'un ensemble activé de la pluralité d'électrodes (104) qui est cartographié spatialement vers une distribution de structures anatomiques sur la base d'un atlas de chemins de conduction spécifiques à la configuration d'ECG et à l'activation de la pluralité d'électrodes (104) ;
la prédiction de substituts de paramètres de fonction cardiovasculaire à partir des données d'ECG et des données de BIA en utilisant un algorithme d'apprentissage profond entraîné, les paramètres de fonction cardiovasculaire étant des paramètres cardiovasculaires d'intérêt traditionnellement dérivés d'au moins l'une parmi l'imagerie, les analyses de sang et les mesures de pression ; et
la délivrance des substituts des paramètres de fonction cardiovasculaire dans un format clinique.

11. Procédé selon la revendication 10, dans lequel la prédiction des substituts des paramètres de fonction cardiovasculaire comprend la génération de données de pléthysmographie d'impédance (IPG) représentatives de la fonction cardiovasculaire au fil du temps à partir des données d'ECG et des données de BIA.

12. Procédé selon la revendication 10 ou la revendication 11, dans lequel la prédiction des substituts des paramètres de fonction cardiovasculaire comprend la génération de données de tomographie par impédance (ITG), incluant des images statiques ou dynamiques d'un cœur et d'un poumon du patient (106), représentatives de la fonction cardiovasculaire et pulmonaire au fil du temps à partir des données d'ECG et des données de BIA.

13. Procédé selon l'une quelconque des revendications 10 à 12, dans lequel la prédiction des substituts des paramètres de fonction cardiovasculaire comprend la prédiction d'un niveau ou d'un proxy d'au moins l'un des paramètres de fonction cardiovasculaire.

14. Procédé selon l'une quelconque des revendications 10 à 13, dans lequel la prédiction des substituts des paramètres de fonction cardiovasculaire comprend la prédiction d'un niveau ou d'un proxy de peptide natriurétique cérébral (BNP) ou de peptide natriurétique cérébral N-terminal (NT-proBNP).

15. Procédé selon l'une quelconque des revendications 10 à 14, comprenant en outre :
la modélisation des modifications des paramètres de la fonction cardiovasculaire et de l'hémodynamique.

16. Procédé selon l'une quelconque des revendications 10 à 15, comprenant en outre :
la modélisation des modifications de paramètres de composition corporelle ; et
la prédiction, sur la base de la modélisation des modifications des paramètres de composition corporelle, d'un niveau ou d'un proxy de concentrations de médicament hydrophile dans un corps du patient (106).
